(19) Europäisches Patentamt
European Patent Office
Office européen des brevets

(11) Veröffentlichungsnummer: **0 214 569 A2**

# (12) EUROPÄISCHE PATENTANMELDUNG

(21) Anmeldenummer: 86111970.9

(22) Anmeldetag: 29.08.86

(51) Int. Cl.⁴: **C12P 7/40**, C12P 41/00

(30) Priorität: 09.09.85 DE 3532026

(43) Veröffentlichungstag der Anmeldung: 18.03.87 Patentblatt 87/12

(84) Benannte Vertragsstaaten: AT BE CH DE FR GB IT LI NL SE

(71) Anmelder: HOECHST AKTIENGESELLSCHAFT
Postfach 80 03 20
D-6230 Frankfurt am Main 80(DE)

(72) Erfinder: Wullbrandt, Dieter, Dr.
Bienerstrasse 29
D-6238 Hofheim am Taunus(DE)
Erfinder: Keller, Reinhold, Dr.
Wiesenweg 5
D-6232 Bad Soden am Taunus(DE)
Erfinder: Schlingmann, Merten, Dr.
Schneidhainer Strasse 32a
D-6240 Königstein/Taunus(DE)

## (54) Verfahren zur Herstellung optisch aktiver alpha-Phenoxypropionsäure und deren Derivate.

(57) Ester von Racematen der α-Phenoxypropionsäure und deren Derivaten werden durch enzymatische Hydrolyse mit Hilfe von Lipasen, Esterasen oder Proteasen nach kurzer Inkubationszeit mit hohen Ausbeuten aufgetrennt.

EP 0 214 569 A2

# Verfahren zur Herstellung optisch aktiver α-Phenoxypropionsäure und deren Derivate

Viele, aus der Literatur bekannte, synthetische Wuchsstoffe und Herbizide oder deren Vorstufen, wie z.B. α-Phenoxypropionsäure, liegen bei ihrer Verwendung oder Weiterverarbeitung in Form von Racematen vor. Häufig ist jedoch eines der optisch aktiven Isomere wirksamer als das andere bzw. als das Racemat. Da es oft wirtschaftlicher ist, die entsprechende Substanz nur in der wirksameren Form einzusetzen, versucht man, die Racemate in ihre Enantiomere aufzutrennen. Eine Möglichkeit der Auftrennung stellt die enzymatische Racematspaltung dar. Cambou und Klibanov bewerten in einem Übersichtsartikel (Biotechnol., Bioeng., Vol 26, 1449, 1984), nach einem Vergleich der Effizienz der verschiedenen enzymatischen Auftrennungen von racemischer p-Chlorphenoxypropionsäure, die enzymatische Hydrolyse als die wohl wirtschaftlichste Trennmethode.

In den europäischen Patentanmeldungen 0 133 033 und 1 133 034 wird die Transformation des L-Enantiomers der α-Phenoxypropionsäure und deren Derivaten in das D-Enantiomer mit Hilfe von Mikroorganismen bzw. deren Enzymen beschrieben. Es können jedoch nur geringe Mengen der L-Verbindung während einer mehrere Tage dauernden Inkubationszeit umgesetzt werden, so daß diese Methode nicht wirtschaftlich erscheint.

Die vorliegende Erfindung bezieht sich auf ein Verfahren, mit dem es überraschenderweise gelungen ist, größere Mengen der Racemate der α-Phenoxypropionsäure und deren Derivate nach kurzer Inkubationszeit mit hohen Ausbeuten aufzutrennen. Die Ester der genannten Racemate werden enzymatisch gespalten, wobei spezifisch nur ein Enantiomer hydrolysiert wird.

Die Erfindung betrifft somit ein Verfahren zur Herstellung optisch aktiver Enantiomere durch enzymatische Racematspaltung, das dadurch gekennzeichnet ist, daß die Verbindung der allgemeinen Formel I,

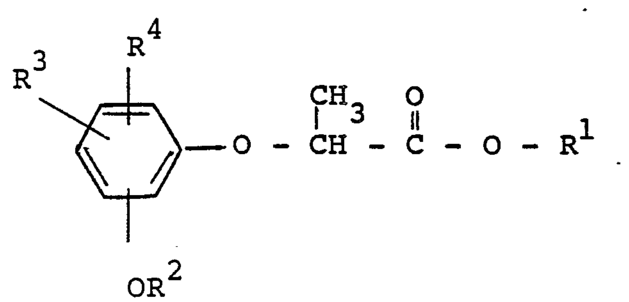

I

in der

a) $R^1$ eine Alkylgruppe mit bis zu 8 C-Atomen, die jeweils mit Hydroxy, Halogen, Alkyl und Nitro substituiert sein können oder eine cyclische Alkylgruppe mit bis zu 6 C-Atomen und

b) $R^2$ Wasserstoff oder eine Alkylgruppe oder eine Arylgruppe oder ein heterozyklisches Ringsystem mit jeweils bis zu 10 C-Atomen, und

c) $R^3$ und $R^4$ unabhängig voneinander Wasserstoff oder Halogen bedeuten, enzymatisch hydrolysiert wird.

Im folgenden wird die Erfindung detailliert beschrieben, bzw. in den Ansprüchen definiert.

Als Ausgangsprodukte dienen die Racemate der α-Phenoxypropionsäure und deren Derivate. Die Veresterung dieser Produkte erfolgt nach an sich bekannten Methoden mit den entsprechenden Hydroxylverbindungen, so daß man zu den obengenannten Estern (I) gelangt.Bevorzugt werden Ester mit niederer Alkylgruppe mit bis zu 5 C-Atomen, insbesondere einer unverzweigten Alkylgruppe mit 1 bis 3 C-Atomen, für die weitere Reaktion verwendet.

Im folgenden erfindungsgemäßen Reaktionsverlauf wird der Ester direkt oder in Kombination mit einem Lösemittel eingesetzt. Hierzu eignen sich inerte organische Lösemittel, in denen die L- und D-Ester löslich sind. Bevorzugt werden Kohlenwasserstoffe, wie Alkane mit einer Kettenlänge von bis zu 10 Kohlenstoffatomen sowie Toluol oder Xylol, Ether, wie Z.B. Di-isopropylether, Methylisobutylether tertiär Butylmethylether und Methylisopropylether, und Ketone, wie z.B. Methylethylketon.

Die enzymatische Hydrolyse kann mit Lipasen, Esterasen und Proteasen durchgeführt werden. Die besten Ergebnisse werden mit einer Lipase aus Candida cylindracea oder α-Chymotrypsin erzielt. Es ist vorteilhaft, das Enzym in fixierter Form einzusetzen. Die Immobilisierung an Copolymerisate, wie sie in den deutschen Offenlegungsschriften 3 404 021 und 3 344 912 beschrieben wird, oder an ein makroporiges Anionenaustauscherharz der Phenol-Formaldehydreihe, das in der japansichen Patentanmeldung Sho 59-183691 genannt wird, hat sich besonders bewährt.

Die Hydrolyse des D-Esters wird mit dem in Puffer gelösten Enzym bzw. enzymbeladenen Träger bei einer Temperatur von 15 bis 40°C, vorzugsweise bei 20 bis 30°C, durchgeführt. Die Reaktion läuft vorteilhaft in einem pH-Bereich von 5 bis 8,5 ab. Das pH-Optimum ist jedoch von dem jeweiligen Enzym und dem eingesetzten Substrat abhängig und kann in kurzen, nicht aufwendigen Vorversuchen bestimmt werden. Die Inkubationszeit beträgt unter den genannten Bedingungen nur mehrere Stunden.

Nach Beendigung der Reaktion wird der Ester der L-Säure mit einem organischen Lösemittel extrahiert. Ist das Racemat schon in Kombination eines Lösemittels zum Reaktionsgemisch gegeben worden, so verwendet man zweckmäßig das gleiche Lösemittel auch zur Extraktion. Weitere geeignete Lösemittel zur Extraktion sind Diethylether sowie halogenierte Kohlenwasserstoffe, wie z.B. Methylenchlorid, Chloroform oder Tetrachlorkohlenstoff.

Die D-Säure kann, nach Ansäuren des verbleibenden wäßrigen Extrakts, ebenfalls durch Extraktion mit einem der o. g. Lösemittel isoliert werden.

Im folgenden wird die Erfindung anhand von Beispielen detailliert erläutert.

Beispiele

Immobilisierung von Lipase aus Candida cylindracea

Beispiel 1

Zu 9 g eines Copolymerisats aus N-Hydroxymethylacylamid, N-Vinylpyrrolidon und N,N'-Methylenbisacrylamid, das gemäß Beispiel 1 der deutschen Offenlegungsschrift 3 404 021 hergestellt wird, gibt man 86 ml 0,1-molaren Phosphatpuffer mit einem pH-Wert von 7,5 sowie 1,0 g Lipase aus Candida cylindracea. Man inkubiert unter Rühren über 72 Stunden bei Raumtemperatur. Nach der Fixierung werden die Perlen mit 0,5-molarer Kochsalzlösung, destilliertem Wasser und oben genannter Phosphatpufferlösung gründlich gewaschen.

Die anschließende Bestimmung der Enzymaktivität mit dem im folgenden beschriebenen Testsystem ergab: 11 U/g Träger

Testsystem: 4 g D,L-2-(p-Hydroxyphenoxy)-propionsäureethylester in 30 ml 0,1-molarem Phosphatpuffer (pH 6,5) werden durch Zugabe von 2 g enzymbeladenem Träger bei 30°C hydrolisiert. 1 U entspricht der Menge Enzym, die zur Umsetzung von 1 µMol Substrat pro Minute benötigt wird.

Beispiel 2

Zu 10 g eines Copolymerisats aus Vinylacetat und Divinylethylenharnstoff, das gemäß Beispiel 16 der deutschen Offenlegungsschrift 3 344 912 hergestellt wird, gibt man 10 g Tetramethylendiamin in 300 ml Dimethoxyethan und erwärmt 4 Stunden auf 80°C. Nach dem Erkalten wird der Träger mit destilliertem Wasser gewaschen. Anschließend wird das Copolymerisat in 150 ml 25 %iger wäßriger Glutardialdehydlösung aufgenommen und 2 Stunden bei Raumtemperatur gerührt.

Der Träger wird danach abfiltriert, gründlich mit destilliertem Wasser gewaschen, in eine Lösung von 1,5 g Lipase aus Candida cylindracea (Firma Sigma) in 150 ml 0,1-molarem Phosphatpuffer (pH 7,5) eingetragen und 16 Stunden bei Raumtemperatur gerührt. Der Träger wird wieder abfiltriert und mit entsalztem Wasser und 0,5-molarer Kochsalzlösung gewaschen. Die anschließende Bestimmung der Enzymaktivität mit dem Testsystem gemäß Beispiel 1 ergab 6 U/g Träger.

Beispiel 3

Gemäß Beispiel 1 der japanischen Patentanmeldung Sho 59-183691 (siehe Anhang) wird Lipase aus Candida cylindracea an ein makroporiges Anionenaustauscherharz der Phenolformaldehydreihe adsorbiert. Man erhält eine Enzymaktivität von 22 U/g Träger.

Enzymatische Racematspaltung

Beispiel 4

In ein Reaktionsgefäß, das mit Innenrührer, pH-Elektrode und automatischer Bürette versehen ist, werden 40 g D,L-2-(p-Hydroxyphenoxy)-propionsäureethylester gegeben und in 300 ml 0,1-molarem Phosphatpuffer (pH 6,5) dispergiert. Durch Zugabe von 1,5 g Lipase aus Candida cylindracea wird die Reaktion gestartet. Es wird über 8 bis 9 Stunden unter Rühren inkubiert, wobei der pH-Wert der Lösung durch Zugabe von 5 N Natronlauge auf 6,5 gehalten wird.

Nach Beendigung der Reaktion wird die Lösung, die Ethyl-L-2-(p-Hydroxyphenoxy)-propionat und die entsprechende D-Säure enthält, bei Raumtemperatur 3 mal mit 100 ml Diethylether ausgeschüttelt. Der getrocknete Extrakt wird eingeengt und der Rückstand im Vakuum bei 0,6 mbar und 135°C destilliert.

Der verbleibende wäßrige Extrakt wird mit konzentrierter Salzsäure auf einen pH-Wert von 1,0 eingestellt und ebenfalls 3 mal mit 100 ml Diethylether extrahiert. Die getrocknete Etherphase wird auf 50 ml eingeengt und mit n-Hexan versetzt, so daß die D-Säure auskristallisiert.

Ergebnis:

L-2-(p-Hydroxyphenoxy)-propionsäureethylester

Ausbeute: 19 g, 95 % d.Th.

optischer Drehwert: $[\alpha]_D^{25}$ -40,8° (c = 2,03 in $CHCl_3$)

D-2-(p-Hydroxyphenoxy)-propionsäure:

Ausbeute: 14 g, 80 % d.Th.

optischer Drehwert: $[\alpha]_D^{20}$ + 41,3° (c = 2,0 in $C_2H_5OH$)

Beispiel 5

Man verfährt gemäß Beispiel 4 unter Verwendung von 20 g fixierter Lipase aus Beispiel 1 bis 3. Ausbeuten und optische Drehwerte sind analog Beispiel 4.

Beispiel 6

In ein Reaktionsgefäß, das mit Innenrührer, pH-Elektrode und automatischer Bürette versehen ist, werden 4,5 g D,L-2-(p-Hydroxyphenoxy)-propionsäureethylester in 50 ml 0,1-molarem Phosphatpuffer (pH 6,4) dispergiert. Man hydrolysiert durch Zugabe von 15 ml immobilisiertem α-Chymotrypsin, das analog Beispiel 1 der deutschen Patentanmeldung P 3 438 189.9 hergestellt wird. Nach einem Umsatz von 42 % wird die Reaktion abgebrochen.

Ergebnis:

L-2-(p-Hydroxyphenoxy)-propionsäureethylester:

optischer Drehwert: $[\alpha]_D^{25}$ -37,3° (c = 2,2 in $CHCl_3$)

D-2-(p-Hydroxyphenoxy)-propionsäure:

optischer Drehwert: $[\alpha]_D^{25}$ + 42,3° (c = 2,1 in $C_2H_5OH$)

Beispiel 7

15 g D,L-2-(p-Methoxyphenoxy)-propionsäureethylester in 150 ml 0,1-molarem Phosphatpuffer (pH 7,7) wird mit 9 g Träger, der gemäß Beispiel 3 hergestellt wird (und eine Enzymaktivität von 12 U/g Träger hat, bezogen auf das o. g. Substrat), wie in Beispiel 4 enzymatisch gespalten. Die Isolierung des L-Esters und der D-Säure wird ebenfalls analog Beispiel 4 durchgeführt.

Ergebnis:

L-2-(p-Methoxyphenoxy)-propionsäureethylester:

Ausbeute: 7 g

optischer Drehwert: $[\alpha]_D^{25}$ -40,97° (c = 1,932 in $CHCl_3$)

D-2-(p-Methoxyphenoxy)-propionsäure:

Ausbeute: 4,9 g

optischer Drehwert: $[\alpha]_D^{25}$ + 30,16° (c = 2,1 in $C_2H_5OH$)

nach Kristallisation aus Hexan: $[\alpha]_D^{25}$ + 40,3 - (c=1,38; in Ethanol)

Beispiel 8

Man verfährt gemäß Beispiel 4, löst jedoch den D,L-Ester vor Zugabe zu dem Phosphatpuffer in 100 ml tertiär Butylmethylether. Das Ergebnis ist in Beispiel 4 aufgeführt.

Beispiel 9

Man verfährt gemäß Beispiel 7, löst jedoch den D,L-Ester in 100 ml n-Hexan bzw. tertiär Butylmethylether. Das Ergebnis ist in Beispiel 7 aufgeführt.

Beispiel 10:

Man verfährt gemäß Beispiel 7 unter Verwendung von 10 g 2-Methylbutyl-2-phenoxypropionat.

Die Isolierung des L-Esters und der D-Säure wird analog Beispiel 4 durchgeführt.

Der erhaltene L-2-Phenoxypropionsäure-2-methylbutylester hat einen optischen Drehwert von $[\alpha]_D^{20}$ -27,03° (c = 2,168 in Chloroform)

Anhang: Immobilisierung von Lipase aus Candida cylindracea an ein Phenolformaldehydharz gemäß Beispiel der Patentanmeldung Sho 59-183691

3 g Lipase-Trockenpulver aus Candida cylindracea (Sigma) wird bei pH 6,0 in 180 ml 50 mmolarem Phosphatpuffer gelöst. In diese Lösung wurden 30 g makroporöses Anionenaustauscher-Harz der Phenol-Formaldehydreihe (Im Handel befindliches (R)Duolite 17-7) gegeben und bei 30°C 6 h mit 120 Umdrehungen/Minute gerührt.

Anschließend wird der Träger abfiltriert, mit Wasser gewaschen und in 150 ml Glutardialdehydlösung der Konzentration 0,5 (Gew./Vol) % bei pH 6,0 und einer Temperatur von 20°C 3,5 Stunden mit 120 Umdrehungen/Minute gerührt. Danach wird der Träger abfiltriert und mit destilliertem Wasser gewaschen.

## Ansprüche

1. Verfahren zur Herstellung optisch aktiver Enantiomere durch enzymatische Racematspaltung, dadurch gekennzeichnet, daß die Verbindung der allgemeinen Formel I,

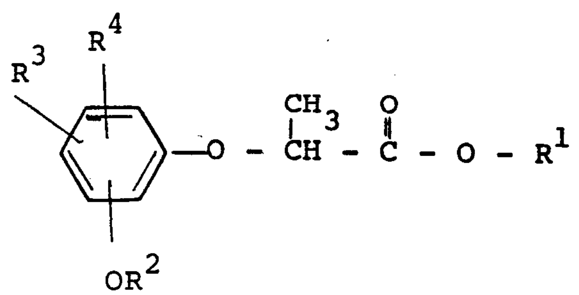

I

in der

a) $R^1$ eine Alkylgruppe mit bis zu 8 C-Atomen, die jeweils mit Hydroxy, Halogen, Alkyl und Nitro substituiert sein können oder eine cyclische Alkylgruppe mit bis zu 6 C-Atomen und

b) $R^2$ Wasserstoff oder eine Alkylgruppe oder eine Arylgruppe oder ein heterozyklisches Ringsystem mit jeweils bis zu 10 C-Atomen, und

c) $R^3$ und $R^4$ unabhängig voneinander Wasserstoff oder Halogen

bedeuten, enzymatisch hydrolysiert wird.

2. Verfahren nach Anspruch 1, dadurch gekennzeichnet, daß die Hydrolyse mit einer Esterase oder Lipase oder Protease durchgeführt wird.

3. Verfahren nach Anspruch 2, dadurch gekennzeichnet, daß eine Lipase aus Candida cyclindracea oder α-Chymotrypsin verwendet wird.

4. Verfahren nach Anspruch 2, dadurch gekennzeichnet, daß die Hydrolyse mit einem immobilisierten Enzym durchgeführt wird.

5. Verfahren nach Anspruch 1, dadurch gekennzeichnet, daß die Verbindung der allgemeinen Formel I dem Reaktions gemisch in Kombination miat einem organischen Lösemittel zugegeben wird.

6. Verfahren nach Anspruch 1, dadurch gekennzeichnet, daß die Hydrolyse bei einer Temperatur von 15 bis 40°C durchgeführt wird.

7. Verfahren nach Anspruch 6, dadurch gekennzeichnet, daß die Hydrolyse bei einer Temperatur von 20 bis 30°C durchgeführt wird.